# EUROPEAN PATENT APPLICATION

(11) **EP 1 617 214 A1**
(43) Date of publication of application: **18.01.2006**
(21) Application number: 04729294.1
(22) Date of filing: 23.04.2004
(51) Int. Cl.: G01N 33/48

(54) **METHOD OF CREATING DISEASE PROGNOSIS MODEL, METHOD OF PREDICTING DISEASE PROGNOSIS USING THE MODEL, DEVICE FOR PREDICTING DISEASE PROGNOSIS USING THE MODEL, ITS PROGRAM, AND RECORDING MEDIUM**

(30) Priority: 23.04.2003 JP 2003118496
(71) Applicant: Eisai Co. Ltd., Tokyo 112-8088 (JP); Yatsuhashi, Hiroshi, Nagasaki 856-0046 (JP); Akiyama, Masanori, Suginami-ku, Tokyo 1670054 (JP)
(72) Inventor: YATSUHASHI, Hiroshi, Omura-shi, Nagasaki 8560046 (JP); AKIYAMA, Masanori, Tokyo 1670054 (JP); MATSUMOTO, Takeharu, Omura-shi, Nagasaki 8560046 (JP)
(74) Representative: HOFFMANN EITLE
(86) International application number: PCT/JP2004/005915
(87) International publication number: WO 2004/095021

(57) **Abstract**

The present invention relates to a disease prognosis prediction modeling method for preparing a model for predicting the prognosis of a specified disease from clinical laboratory test values for the disease by means of a computer, the method comprising the steps of: inputting a plurality of actually measured clinical laboratory test values for the disease and actual measured values of the prognoses into the computer; processing these values by a data mining method to determine one or a plurality of clinical laboratory test items which have an influence on the prognosis of the disease; determining a priority of the items with respect to the prognosis in a case where there are a plurality of the items; and establishing a judgment routine in which correlation of the plurality of clinical laboratory test items and the clinical laboratory test value ranges of the test items with the predicted value of the prognosis is stipulated on the basis of the priority, wherein the judgment routine is used as the model.

## Description

### Technical Field

The present invention relates to a model for predicting the prognosis of a disease, and a prediction method utilizing this model.

### Background Art

In Japan, there are approximately 2,000,000 HCV carriers and approximately 1,000,000 HBV carriers. Some of these carriers progress over the long term to chronic hepatitis and hepatic cirrhosis, and these carriers die with complications of liver cancer.

The first diagnosis of liver disease and liver cancer is an image diagnosis. In this case, however, the cost is high, and special instruments and techniques are required. Blood tests, which are one of clinical laboratory tests, are also used in the diagnosis of such disorders. However, these are merely an aid to the image diagnosis.

One of the blood test findings for liver disease and liver cancer is the measurement of PIVKA. It has been discovered that PIVKA appears in the blood in the case of liver disease, and PIVKA-II appears with high frequency in hepatocellular carcinoma patients showing negative for α-hetoprotein, which has been regarded as a good marker for hepatocellular carcinoma. Accordingly, PIVKA-II has become established as a tumor marker for liver cancer.

HCV, HBV and progressive type liver cancer are chronic disorders, and the main part of the therapy for such disorders is an extension of the prognosis. Conventionally, the prediction of prognosis and expected survival years for patients suffering from such liver disorders, have been based on the personal experience of the physician as determined from the results of image diagnosis. Accordingly, an accurate prediction of prognosis (including expected survival years) has been difficult.

In view of the above, it is an object of the present invention to construct a model for an accurate prediction of the prognosis of patients from clinical laboratory test values, and to provide a method for accurately predicting the prognosis of patients on the basis of this model.

### Disclosure of Invention

The present inventor has constructed an already-described model by analyzing blood test findings and prognoses of the disease, e. g., actual measurement values of survival years, using data processing methods such as a data mining method and the like. The data mining method is an advanced information analysis system which promotes important decision-making support by analyzing past data and discovering regularities in this data. This method was established in the financial business field, and has been widely introduced. Since conventional statistical methods are methods that verify hypotheses using a limited number of samples, there are difficulties in terms of completeness and speed. In the case of the data mining method, however, a high-speed search is made in a comprehensive manner from a large amount of data, so that a precise analysis is possible.

Actual measurement values of clinical laboratory test findings and prognoses (e. g., survival years) are compared, the priority of clinical laboratory test items involved in the prognosis of diseases is determined, and judgment branch routines are constructed in which clinical laboratory test items that have a higher priority are placed on the upstream side. Then, predictions of prognoses (i. e., the certainty of prognosis) can be obtained by applying the measured values of clinical laboratory test items to these judgment branch routines.

The present invention was devised on the basis of such findings, and provides a disease prognosis prediction modeling method for preparing a model for predicting the prognosis of the disease from clinical laboratory test values for the disease by means of a computer, the method comprising the steps of: inputting a plurality of actually measured clinical laboratory test values for the disease and actually measured values of the prognoses into the computer; processing these values by a data mining method to determine one or a plurality of clinical laboratory test items which have an influence on the prognosis of the disease; determining a priority of the items with respect to the prognosis in a case where there are a plurality of the items; and establishing a judgment routine in which correlation of the plurality of clinical laboratory test items and the clinical laboratory test value ranges of the test items with the predicted value of the prognosis is stipulated on the basis of the priority, wherein the judgment routine is used as the model.

In a preferred aspect of the present invention, the above-mentioned judgment routine is a decision tree in which a plurality of chance nodes are taken as the clinical laboratory test items and the clinical laboratory test measurement value ranges, and a plurality of prognosis prediction values corresponding to the chance nodes are taken as terminal nodes. Further, the prognosis of the disease can be predicted from a disease name and the plurality of clinical laboratory test measurement values on these bases of the already-described judgment routine.

Another invention relates to a disease prognosis prediction method for predicting the prognosis of the disease from clinical laboratory test data using a computer, the method comprising the steps of: storing the judgment routine according to claim 1 or 2 in a computer: inputting a name of the disease which is an object of the prognosis prediction and clinical laboratory test measurement values for the disease into the computer; and determining a predicted value of the prognosis of the disease using the input values on the basis of the judgment routine. Further, still another invention relates to a disease prognosis prediction device which predicts the prognosis of the disease from clinical laboratory test values, and which comprises a computer, wherein the computer comprises a memory that stores the judgment routine; input means that inputs a name of the disease which is an object of the prognosis prediction and clinical laboratory test measurement values for the disease; prognosis prediction value acquisition means that determines the prognosis prediction value for the disease by applying the input values to the judgment routine; and display processing means that displays the prognosis prediction value thereon.

Other invention relates to a program which causes a computer to execute the respective means described above, and which is readable by the computer, and a storage medium in which this program is stored.

One of the objects to which the present invention is applied is a liver disease, wherein the clinical laboratory test item with the highest priority described above is PIVKA. The above-mentioned judgment routine is a decision tree in which a plurality of chance nodes are taken as the clinical laboratory test items and clinical laboratory test measurement value ranges, and a plurality of prognosis prediction values corresponding to these chance nodes are taken as terminal nodes. The chance nodes of the decision tree include patient information. Furthermore, the present invention is a data group which forms the decision tree. This data can be recorded on a CD, DVD, HD or the like used as a storage medium.

Furthermore, the present invention relates to a method for predicting a prognosis relating to a disease of a certain patient from test values for current clinical laboratory test items for the disease of the patient by means of a model in which statistical processing is performed on the basis of the relationship between test results, which relate to a plurality of patients, obtained for a clinical laboratory test item indicating the disease, and the actual prognoses of the disease for the respective patients. One example of this test item is a test item relating to PIVKA. The method is devised so that the priority of the clinical test items is determined each time in the process of the judgment routine. The above-mentioned disease is a disease relating to the liver, and the highest chance node is set at a critical value relating to the clinical laboratory test value of PIVKA. A PIVKA reference value is set for each year of survival years when survival predictions in which PIVKA is the node with the highest priority are performed on the basis of the model for each year of survival years.

According to the present invention, it has been found that PIVKA is the clinical laboratory test item with the highest priority (the diagnostic marker of first choice) in predicting the prognosis of liver diseases. Accordingly, the present invention provides a method for predicting prognosis of the disease from actual patient data by a procedure in which patient data (age, body weight, sex, image data such as MRI or the like, clinical laboratory measurement values, blood test findings and the like) are sorted in accordance with the degree of the influence that such data has on prognosis of the disease.

### Brief Description of Drawings

Fig. 1 is a block diagram of the hardware that is used to implement the method according to the present invention;
Fig. 2 is a system diagram showing the decision tree that is obtained in the present invention; and
Fig. 3 is a functional block diagram of the device according to the present invention.

### Best Mode for Carrying Out the Invention

456 patients dying of liver disease during the period 1990 to 2002 (325 male patients, 131 female patients, mean age: 64 years, ranging in age from 25 to 92 years) were used as subjects. Among these patients, the diagnosis at the time of death was liver cancer in 346 cases, chronic cirrhotic liver failure in 59 cases, acute liver failure in 14 cases, and other problems in 37 cases.

Patient information and information relating to blood test findings (approximately 25,000 findings per item for a plurality of items including Alb, ALT, LDH, CHO, PIVKA and the like) were analyzed by use of a "DB2 Intelligent Miner" (commercial name) which is a data mining tool manufactured by Nippon IBM Co., and a decision tree was prepared as a one-year survival judgment model for judging whether or not the patients survived for one year from the time of testing.

The decision tree is constructed from nodes and links. Each of nodes corresponds to classifying attributes, and the links which connect the nodes with the lower nodes correspond to attribute values. Classes that are classified by the link attribute values from the highest node are expressed in the lower nodes.

For example, attributes are constructed from the specifications of clinical laboratory test items and individual patient data items, and specifications of numerical value ranges of these items (defined by conditional symbols such as =, >, ≥, <, ≤, ≠, ≅ and the like).

The higher nodes and lower nodes are determined on the basis of priority, ranges are defined in the links, and the certainty of prognosis predictions is defined in the terminal nodes. According to current findings, it was found that the highest node relates to PIVKA in cases of liver cancer or hepatitis. Accordingly, in case where the prognosis (survival year) for liver cancer is predicted, PIVKA blood test findings constitute the marker of first choice.

Other items are as follows: test date, date of death, age at time of testing, age at time of death, prognosis at time of testing, number of days from testing to death, sex, virus type, name of disease, TP: total protein, ALB: albumin, GLB: globulin, A/G: ratio of albumin to globulin, TTT: thymol, ZTT: Kunkel's test, T-BIL: total bilirubin, D-BIL: bilirubin fraction, GOT, GPT, LDH: lactate dehydrogenase, ALP: alkaline phosphatase, γGTP: gamma-GTP, LAP: leucine aminopeptidase, CH-E: cholinesterase, BUN: urea nitrogen in urine, CREA: creatinine, URICA, NA: sodium, CL: chlorine, K: potassium, CA: calcium, T-CHO: total cholesterol, AFP: α-fetoprotein, PIVKA-II.

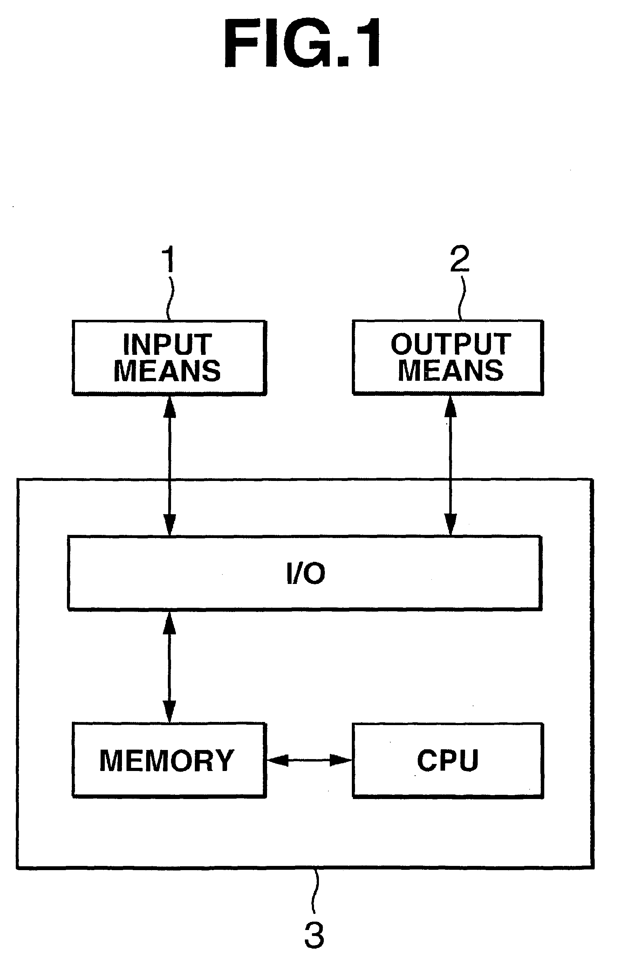

Fig. 1 is a block diagram of the hardware used to realize the prognosis prediction modeling method and device according to the present invention. This device is constructed from a micro-computer. The micro-computer comprises input means (keyboard or the like) 1, output means (liquid crystal monitor or the like) 2, and a computer main body (memory, CPU, I/O and the like) 3. A data mining program is stored in the memory.

Here, a model was prepared as follows: namely, in cases where the condition of (PIVKA > 8255 mAU/ml) is satisfied at the time of testing as a result of the above-described information being input into the main body of the invention and analyzed, mortality occurs within one year with a probability of 93.9%, and when the two conditions of (1034 < PIVKA < 8255) and (AFP > 1215 ng/ml) are satisfied, mortality occurs within one year with a probability of 91.7%. On the other hand, when the three conditions of (PIVKA < 1034), (CHO > 102 mg/dl) and (AFP < 531.5) are satisfied, the patient survives for one year or longer with a probability of 85.5%.

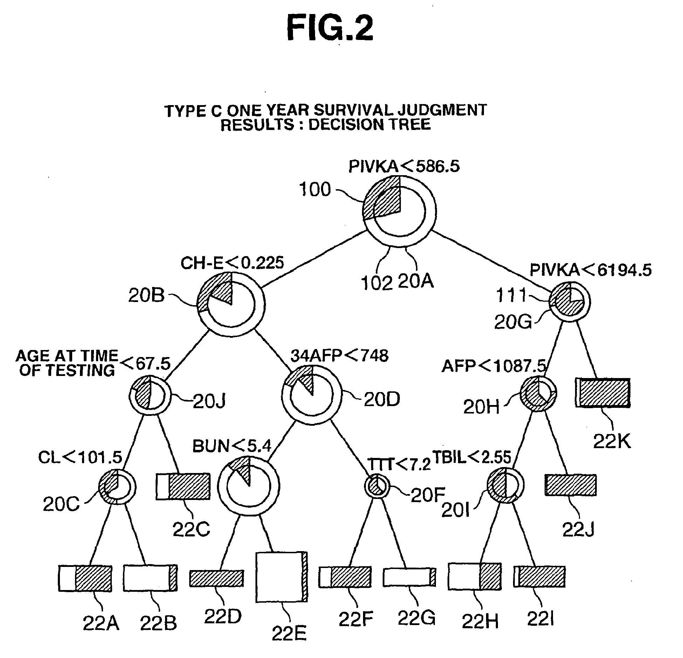

This model is constructed from the decision tree shown in Fig. 2. In Fig. 2, each block (20) indicated by circular shapes represents chance nodes, and the each block indicated by square shapes represents terminal nodes (22). These nodes are respective pluralities of nodes (20A, 20B ...., 22A, 22B .....), and the tree branches from one chance node to other chance nodes or the terminal nodes.

The size of the circle of each chance node corresponds to N (the number of patients), and the region indicated by the shaded part within each circle (for example: 100 of the chance node 20A) indicates the proportion of N with a survival of less than one year, while the region that is not shaded (for example: 102 of the chance node 20A) indicates the proportion of N with a survival exceeding one year.

Among the routes that branch from one chance node to other chance nodes or terminal nodes, the routes on the left side indicate an affirmative with respect to the comparative value of the chance node, while the routes on the right side indicate a negative. For example, in cases where the condition of PIVKA < 586.5 mgAU/ml of the chance node 20A is affirmed, the processing proceeds to the chance node 20B; when this is denied, the processing proceeds to the chance node 20G.

Since the above terminal nodes are nodes that indicate the proportions of survival for one year and mortality within one year, these mark the survival probability within a one year period. Note that in the double circle of each chance node, the proportion of shading / lack of shading on the inside circle corresponds to the proportion of persons surviving or not surviving for one year in this chance node, the proportion of shading / lack of shading in the outside circle corresponds to the proportion of persons surviving or not surviving for one year in the chance node located immediately upstream, and the number obtained by multiplying these proportions is the proportion of survival for one year / mortality within one year according to the judgment of this event.

When actual clinical data and patient data (age and the like) were analyzed along with the prognoses (mortality or survival after one year) by means of the data mining method, a decision tree of the type shown in Fig. 2 was obtained. As is clear from this Fig. 2, in this liver disease / liver cancer prognosis prediction model, the marker of first choice which has the highest priority is the measured value of PIVKA.

Next, the prognosis prediction method and device will be described. This method and device are realized using the same hardware as in Fig. 1. A program corresponding to the above-described decision tree is stored in the memory of the micro-computer main body.

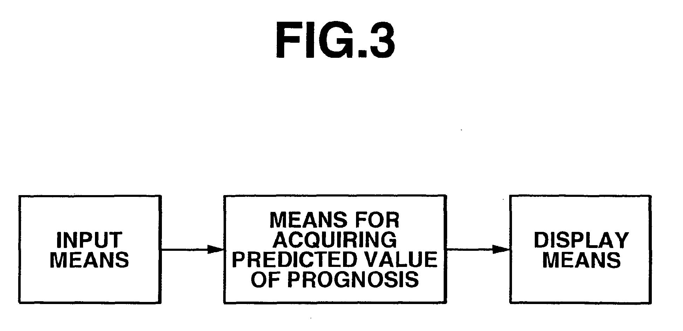

Fig. 3 is a functional block diagram which illustrates the operation of the micro-computer main body. This micro-computer main body is constructed from input means which inputs the name of the disease that is the object of prognosis (type C hepatitis) and clinical laboratory test measurement values (the above-described items) for the patient in question; prognosis prediction value acquisition means which determines the predicted value of the prognosis of the disease by applying these input values to the judgment routine (decision tree); and display processing means (liquid crystal monitor) that is used to display this predicted value of the prognosis on display means.

The prognosis prediction method using this decision tree will be described. Patient information such as the patient's name, patient ID, patient's sex, patient's age and the like, and various clinical laboratory test values, are input using the abovementioned input means of the micro-computer. The CPU of the micro-computer main body temporarily stores this input data in a work RAM which is a part of the memory, and applies the program of the decision tree shown in Fig. 2 to this stored data.

Here, a case will be described in which the prognosis of type C hepatitis is actually predicted using the measured data for a certain patient. The patient data and clinical test findings are input into the computer main body from the input means of the computer. The CPU of the computer main body performs survival prediction processing in accordance with a program corresponding to the decision tree in the memory.

If PIVKA < 586.5 (units omitted; same below) is affirmed in the chance node 20A, the processing proceeds to the chance node 20B. Then, if it is affirmed that CH-E < 0.225, the processing proceeds to the chance node 20J. If the age at the time of testing is less than 67.5 in the chance node 20J, the processing proceeds to the chance node 20C, and a judgment is made as to whether or not CL < 151.5. If this is affirmed, the processing proceeds to the terminal node 22A. If not, the processing proceeds to the terminal node 22B. In the terminal node 22A, the survival after one year (survival of one year or greater) is approximately 70%, while in the terminal node 22B, this survival after one year is approximately 10%. The judgment route of the decision tree is searched from the blood test findings for the patient, and the probability of survival after one year is determined when the corresponding terminal node is reached. This constitutes the predicted value of the prognosis.

A plurality of judgment routines such as a decision tree for judging the two-year survival for type C hepatitis, a decision tree for judging the five-year survival for type C hepatitis and the like can be prepared as decision trees. This can also be expanded to type B hepatitis and other diseases. The predicted value of the prognosis (survival probability) for each disease and survival of each year can be calculated by means of the prognosis prediction method and device according to the present invention by executing all of the judgment routines for a certain patient.

It was confirmed by a procedure using the data mining described here that the absolute values of liver cancer tumor markers and liver reserve function contribute to the survival period of liver disease patients. Besides using a decision tree, it would also be possible to prepare a prognosis prediction model of expected survival years using occasional test values in an analysis using a radial basis function (RBF) or a neural network.

When the predictions were restricted to liver cancer patients, and predicted values of prognoses were determined in the order of half-year survival, one year survival, two year survival on the basis of the above-mentioned model, PIVKA was extracted as the most important factor in all cases, and the respective reference values of 2028 mAU/ml, 1035 mAU/ml and 502 mAU/ml were determined. Accordingly, it was confirmed that a survival prognosis of six months can be predicted if the PIVKA value is 2000 mAU/ml, a survival prognosis of one year can be predicted if the PIVKA value is 1000 mAU/ml, and a survival prognosis of two years can be predicted if the PIVKA value is 500 mAU/ml. Furthermore, these reference values are not limited to these specific values, but may be appropriately altered. Speaking roughly from these reference values, it is likely that the length of survival years and the PIVKA reference value are in an inversely proportional relationship.

The natural course and prognosis of the diseases can be estimated by the analysis using data mining, so that the present model makes a great contribution to the selection of treatment methods for the liver disease patients and the liver cancer patients, such as the application of transplant therapy and the like.

It should be noted that in the model shown in Fig. 2, although clinical test items other than PIVKA were also used in the chance nodes, it was confirmed that even when PIVKA is used alone, predictions of the survival year in liver cancer show substantial agreement with actual results.

## Claims

1. A disease prognosis prediction modeling method for preparing a model for predicting the prognosis of a specified disease from clinical laboratory test values for the disease by means of a computer, the method comprising the steps of:
inputting a plurality of actually measured clinical laboratory test values for the disease and actual measured values of the prognoses into the computer;
processing these values by a data mining method to determine one or a plurality of clinical laboratory test items which have an influence on the prognosis of the disease;
determining a priority of the items with respect to the prognosis in a case where there are a plurality of the items; and
establishing a judgment routine in which correlation of the plurality of clinical laboratory test items and the clinical laboratory test value ranges of the test items with the predicted value of the prognosis is stipulated on the basis of the priority,
wherein the judgment routine is used as the model.

2. The disease prognosis prediction modeling method according to Claim 1, wherein the judgment routine is a decision tree in which a plurality of chance nodes are taken as the clinical laboratory test items and the clinical laboratory test measurement value ranges, and a plurality of prognosis prediction values corresponding to the chance nodes are taken as terminal nodes.

3. A method for predicting the prognosis of a disease from a disease name and the plurality of clinical laboratory test measurement values on the basis of the judgment routine according to claim 1 or claim 2.

4. A disease prognosis prediction method for predicting the prognosis of the disease from clinical laboratory test data using a computer, the method comprising the steps of:
storing the judgment routine according to claim 1 or 2 in a computer;
inputting a name of the disease which is an object of the prognosis prediction and clinical laboratory test measurement values for the disease into the computer; and
determining a predicted value of the prognosis of the disease using the input values on the basis of the judgment routine.

5. A disease prognosis prediction device which predicts the prognosis of the disease from clinical laboratory test values, and which comprises a computer, wherein the computer comprises a memory that stores the judgment routine according to Claim 1 or 2; input means that inputs a name of the disease which is an object of the prognosis prediction and clinical laboratory test measurement values for the disease; prognosis prediction value acquisition means that determines the prognosis prediction value for the disease by applying the input values to the judgment routine; and display processing means that displays the prognosis prediction value thereon.

6. A computer program which causes a computer to execute the respective means according to claim 5, and which is readable by the computer.

7. A storage medium in which the program according to claim 6 is stored.

8. The method according to claim 2, wherein the disease comprises a liver disease, and the clinical laboratory test item with the highest priority comprises PIVKA.

9. The method according to claim 3 or 4, wherein the judgment routine is a decision tree in which a plurality of chance nodes are taken as the clinical laboratory test items and clinical laboratory test measurement value ranges, and a plurality of prognosis prediction values corresponding to the chance nodes are taken as terminal nodes.

10. The method according to claim 9, wherein the chance nodes of the decision tree comprises patient information.

11. A data group which forms the decision tree according to claim 2.

12. A method for predicting a prognosis relating to a disease of a certain patient from test values for current clinical test items for the disease of the patient by means of a model in which statistical processing is performed on the basis of the relationship between test results, which relate to a plurality of patients, obtained for a clinical test item indicating the disease, and the actual prognoses of the disease for the respective patients.

13. The method according to claim 12, wherein the clinical test item relates to PIVKA.

14. The method according to claim 1 or 4, wherein the priority of the clinical test items is determined each time in the process of the judgment routine.

15. The method according to claim 1 or 4, wherein the disease relates to a liver disease, and the highest chance node is set at a critical value relating to the clinical test value of PIVKA.

16. The method according to claim 13, wherein PIVKA reference value is set for each year of survival years when survival predictions in which PIVKA is the node with the highest priority are performed on the basis of the model for each year of survival years.
